Europäisches Patentamt

(19) European Patent Office    (11) Publication number: **0 218 128**
Office européen des brevets                                **B1**

(12)                    EUROPEAN PATENT SPECIFICATION

(45) Date of publication of the patent specification:    (51) Int. Cl.⁵: **A61N 1/05, A61B 17/11**
   **12.12.90**

(21) Application number: **86112878.3**

(22) Date of filing: **18.09.86**

(54) **Vein suture collar.**

(30) Priority: **18.09.85 US 776859**    •    (73) Proprietor: **TELECTRONICS N.V., De Ruyterkade 581 P.O. Box 837, Willemstad Curaçao(AN)**

(43) Date of publication of application:
   **15.04.87 Bulletin 87/16**    (72) Inventor: **Milijasevic, Zoran, 12 Lesley Close, Elanora Heights 2101(AU)**
                                       Inventor: **Stewart, Sue, 46 Dillon Street, Paddington 2021(AU)**

(45) Publication of the grant of the patent:
   **12.12.90 Bulletin 90/50**

                                       (74) Representative: **Patentanwälte Grünecker, Kinkeldey, Stockmair & Partner, Maximilianstrasse 58, D-8000 München 22(DE)**

(84) Designated Contracting States:
   **DE FR GB IT**

(56) References cited:
   **GB-A- 2 090 143**
   **GB-A- 2 161 708**
   **US-A- 3 730 187**
   **US-A- 4 516 584**
   **US-A- 4 553 961**
   **US-A-44 374 75**

ACTORUM AG

## Description

The present invention relates to vein suture collar in accordance with the generic clause of Claim 1.

## DESCRIPTION OF THE PRIOR ART:

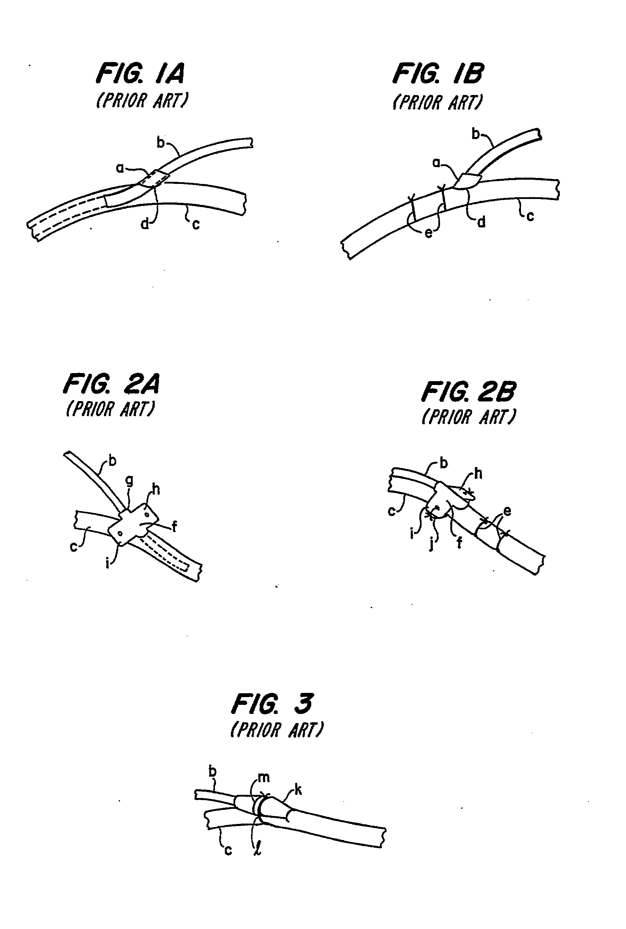

It is well known that damage can occur to both polyurethane and silicone electrode leads if a suture is tightly tied around the body of the electrode lead. To avoid damage to the lead body, manufacturers have developed a variety of suture collars. There are three basic types of suture collars available on the market. The simplest, which is depicted in Figures 1A and 1B, is a silicone tube (depicted by the letter "a" in the figures) which fits over electrode lead body "b" and is partially inserted into vein "c" at the lead entry site "d". Sutures "e" are then tied over the vein, sleeve and lead body. Although not shown, these collars generally have a longitudinal slit to facilitate installation over the lead body.

A slightly more sophisticated collar has a sleeve with a butterfly collar portion which can be anchored to the surrounding tissue. As depicted in Figures 2A and 2B, collar "f" includes butterfly portion "g" with wings "h", "i" which can be folded around vein "c" (Figure 2B) and tied by suture "j".

The third type of collar is a sleeve with a groove formed in the collar exterior surface. As is depicted in Figure 3, a collar "k" of this type sits on the vein and is anchored by tying suture around groove and vein "c".

In US-A 4 516 584 a vein suture collar is described comprising a cylindrical member having a through-bore along the longitudinal axis thereof and two circumferential grooves formed in the exterior surface of the cylindrical member and spaced longitudinally.

A vein suture collar in accordance with the generic clause of Claim 1 is known from US-A 4 437 475 comprising a cylindrical member having a through-bore along the longitudinal axis thereof, a circumferential groove formed in the exterior surface of the cylindrical member and a plurality of raised web areas formed on the interior surface at the end of the cylindrical member and extending radially into the through-bore.

Problems with conventional suture collars stem from the need to be able to slide the collar along the electrode lead into place at the vein entry site while still providing an anchor for the electrode lead. The bore of the collar typically is made only slightly larger than the outer diameter of the lead. However, small variations in either the collar inside diameter or the lead outside diameter can result in a situation where excessive interference is present and undue force is required to slide the collar, leading to possible damage to the lead, or a situation where the collar is too loose and no firm anchor is provided. Also, in either case the sutures can cut the vein if too much tension is applied, especially in the latter situation if the surgeon attempts to constrict the collar by tightening the sutures.

The object of the invention is to provide a vein suture collar which ensures a firm anchor of a electrode inserted in a vein.

The object of the invention is solved with a vein suture collar having the features specified in Claim 1.

It is preferred that the raised web areas are angularly spaced about the interior surface of the member.

It is also preferred that the collar include a slit extending longitudinally along the entire length of the sleeve and radially through the thickness of the sleeve, and that the sleeve is resiliently spreadable at the slit to enclose the vein and the inserted electrode lead.

It is still further preferred that the cylindrical sleeve is made of a flexible material.

## BRIEF DESCRIPTION OF THE DRAWINGS

Figs. 1A and 1B are schematic representations of a conventional suture collar;

Figs. 2A and 2B are schematic representations of another conventional suture collar;

Fig. 3 is a schematic representation of yet another conventional suture collar;

Fig. 4 is a schematic view in partial longitudinal cross section of a suture collar made in accordance with the present invention;

Fig. 5 is a schematic end view of the suture collar shown in Fig. 4; and

Fig. 6 shows a variant of the suture collar depicted in Fig. 4.

## DESCRIPTION OF THE PREFERRED EMBODIMENT

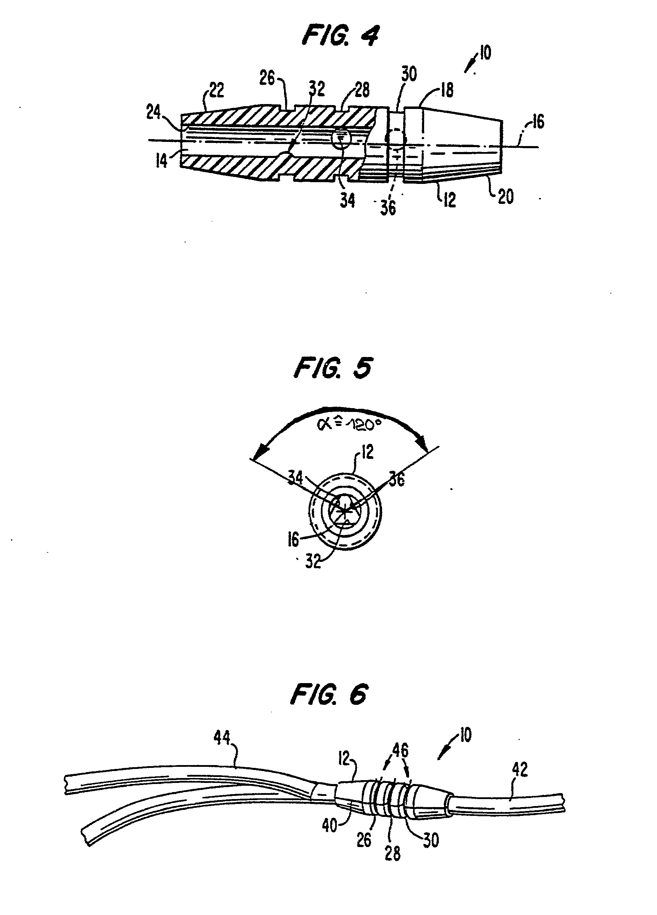

Reference will now be made in detail to the present preferred embodiment of the invention, an example of which is illustrated in Figs. 4-6 of the accompanying drawings which show a suture collar designated generally by the numeral 10 made in accordance with the present invention.

In accordance with the present invention, the vein suture collar comprises a cylindrical member having a through-bore along the longitudinal axis thereof. As embodied herein, and with initial reference to Fig. 4 of the drawings, suture collar 10 includes cylindrical sleeve 12 having through-bore 14 extending along longitudinal axis 16 of sleeve 12. Sleeve 12 can preferably be formed from medical grade silicone or any other flexible, bio-compatible low compression set material. Exterior cylindrical surface 18 of member 12 is tapered at opposing longitudinal ends. In the embodiment shown in Fig. 4, interior cylindrical surface 24 of sleeve 12 has a generally constant diameter along longitudinal axis 16, except for the raised web areas to be discussed hereinafter. The I.D. of the collar generally varies from about 0.25 mm to about 0.35 mm larger than the O.D. of the electrode lead, the variation depending upon the stiffness of the lead, insulation material surrounding the lead (polyurethane or silicone) and whether the lead is unipolar or multipolar.

Further in accordance with the present invention, the vein suture collar further comprises a plurality of circumferential grooves formed in the exterior surface of the member and spaced longitudinally. As embodied herein, and with continued reference to Fig. 4, a total of three circumferential grooves 26, 28, 30 are formed in exterior surface 18 and spaced along longitudinal axis 16. Grooves 26, 28, 30 should be sized to accommodate and capture the suture thread to be used to ligate the vein.

Still further in accordance with the present invention, the vein suture collar includes a plurality of raised web areas formed on the interior surface of the cylindrical member and extending radially into the through-bore. As embodied herein, and with reference to Figs. 4 and 5, a total of three raised web areas 32, 34, 36 are formed on interior surface 24 and extend radially into through-bore 14. It is intended that raised web areas 32, 34, 36 provide the primary contact between collar 10 and the inserted electrode. Thus, for applications where collar 10 is to be used in the manner of conventional suture collars where direct contact between the electrode leads and the collar occurs, the interior diameter 24 of the sleeve 12 can be made larger than the outside diameter of the electrode lead by a margin sufficient to preclude interference between the interior surface 24 and the electrode lead. Concurrently, raised web areas 32, 34, 36 should be sized to provide an interference fit with electrode lead to a degree only to inhibit movement of the collar by gravity alone, while permitting collar 10 to be manually slid along the electrode lead with a small force. The lead-contacting surfaces of raised web areas 32, 34, 36 should be smooth and can preferably be made circular and rounded as depicted in the figures. However, other web area shapes can be used.

Preferably, the web areas are spaced longitudinally along the axis of the cylindrical member and are formed radially beneath respective circumferential suture grooves. As embodied herein, and with reference to Figs. 4 and 5, raised web areas 32, 34, 36 are formed at the same longitudinal locations and radially inward of grooves 26, 28, 30, respectively. This correspondence permits the force exerted by the sutures to be radially directed through the web areas 32, 34, 36 to the electrode lead and permit collar 10 to be flexed between these longitudinal locations.

It is also preferred that raised web areas be angularly distributed on the interior surface about the cylindrical member longitudinal axis. As embodied herein, and with reference to Fig. 5, the three web areas of the preferred embodiment are angularly displaced about axis 16 at about 120° degree intervals as shown by the angle designated alpha. This angular distribution acts to force a plurality of contacts between the lead and the interior surface 24 of collar 10.

The vein suture collar further comprises a slit extending longitudinally along the entire length of the sleeve and radially through the thickness of the sleeve. As a result of the flexible sleeve material, the sleeve can be resiliently spread at the slit to enclose both the vein and inserted electrode lead in a preferred installation mode. As embodied herein and as depicted in Fig. 6, collar 10 can include slit 40 in cylindrical sleeve 12 along the longitudinal direction and through the thickness of sleeve 12. Slit 40 permits collar 10 to be used to protect both vein (designated 42 in Fig. 6) and the electrode lead (designated 44) from being damaged by tightening of sutures 46 in grooves 26, 28, 30. Collar 10 with slit 40 can thus be used in a manner different from conventional suture collars.

Specifically, installation of the collar is accomplished by separating the flexible collar at the slit and enclosing the vein and inserted lead with the separated collar. Following the enclosing step, the sutures are tied around the circumferential grooves to cause the slit edges of cylindrical sleeve to firmly abut. For applications using a slit collar, the raised web areas can be formed to provide a lesser degree of interference with the electrode lead inasmuch as the raised web areas will not contact the electrode lead directly but through the thickness of the vein wall.

It will be apparent to those skilled in the art that various modifications and variations can be made in the vein suture collar of the present invention without departing from the scope of the invention.

**Claims**

1. Vein suture collar comprising:
a cylindrical member (12) having a through-bore (14) along the longitudinal axis (16) thereof;
a circumferential groove formed in the exterior surface of the cylindrical member (12);
a plurality of raised web areas (32, 34, 36) formed on the interior surface (24) of the cylindrical member (12) and extending radially into the through-bore (14); characterized in that
a plurality of the circumferential grooves (26, 28, 30) are spaced longitudinally and in that each of the plurality of raised web areas (32, 34, 36) is located to radially oppose a respective one of the grooves (26, 28, 30).

2. Vein suture collar as in Claim 1 wherein the raised web areas (32, 34, 36) are angularly spaced about the interior surface (24) of the cylindrical member (12).

3. Vein suture collar as in Claim 1 or Claim 2 having three longitudinally spaced grooves (26, 28, 30).

4. Vein suture collar as in any of the preceding claims having three raised web areas (32, 34, 36) spaced along the longitudinal axis (16) of the cylindrical member (12) and angularly spaced about the interior surface (24) of the cylindrical member (12) at about 120 degree intervals.

5. Vein suture collar as in any of the preceding claims wherein each of said raised web areas (32, 34, 36) is generally circular in shape.

6. The vein suture collar as in any of the preceding claims whereby the cylindrical member (12) formed of a resilient material and a slit (40) extending longitudinally along the entire length of the cylindrical member (12) and radially through the thickness of the cylindrical member (12), the cylindrical member (12) being resiliently spreadable at the slit (40) to enclose the vein (42) and inserted electrode lead (44).

7. The vein suture collar as in any of the preceding claims having an inside diameter greater than the outside diameter of the vein (42) with the inserted electrode (44).

**Patentansprüche**

1. Manschette zum Venenvernähen, enthaltend:
einen zylindrischen Körper (12) mit einer Durchgangsbohrung (14) in der Längsachse (16) desselben;
eine Umfangsnut, die in der Außenfläche des zylindrischen Körpers (12) ausgebildet ist;
mehrere erhöhte Stegbereiche (32, 34, 36), die an der Innenfläche (24) des zylindrischen Körpers (12) ausgebildet sind und sich radial in die Durchgangsbohrung (14) erstrecken; dadurch gekennzeichnet, daß
mehrere der Umfangsnuten (26, 28, 30) in Längsrichtung verteilt angeordnet sind und daß jeder der erhöhten Stegbereiche (32, 34, 36) so angeordnet ist, daß er radial einer entsprechenden der Nuten (26, 28, 30) gegenübersteht.

2. Manschette zum Venenvernähen nach Anspruch 1, bei der die erhöhten Stegbereiche (32, 34, 36) im Winkel um die Innenfläche (24) des zylindrischen Körpers (12) verteils sind.

3. Manschette zum Venenvernähen nach Anspruch 1 oder 2 mit drei in Längsrichtung verteilten Nuten (26, 28, 30).

4. Manschette zum Venenvernähen nach einem der vorhergehenden Ansprüche mit drei erhöhten Stegbereichen (32, 34, 36), die in der Längsachse (16) des zylindrischen Körpers (12) verteilt angeordnet und im Winkel um die Innenfläche (24) des zylindrischen Körpers (12) in Intervallen von etwa 120° versetzt angeordnet sind.

5. Manschette zum Venenvernähen nach einem der vorhergehenden Ansprüche, bei der jeder der erhöhten Stegbereiche (32, 34, 36) im wesentlichen kreisförmige Gestalt hat.

6. Manschette zum Venenvernähen nach einem der vorhergehenden Ansprüche, bei der der zylindrische Körper (12) aus einem elastischen Material besteht und ein Schlitz (40) sich in Längsrichtung über die gesamte Länge des zylindrischen Körpers (12) und radial durch die Dicke des zylindrischen Körpers (12) erstreckt, wobei der zylindrische Körper (12) elastisch an dem Schlitz (40) aufspreizbar ist, um die Vene (42) und einen eingesetzten Elektrodendraht (44) zu umschließen.

7. Manschette zum Venenvernähen nach einem der vorhergehenden Ansprüche mit einem Innendurchmesser, der größer als der Außendurchmesser der Vene (42) mit der eingesetzten Elektrode (44) ist.

**Revendications**

1. Gaine pour la suture veineuse comprenant:
un élément cylindrique (12) ayant un trou passant (14) le long de son axe longitudinal (16);
une rainure circonférentielle formée dans la surface externe de l'élément cylindrique (12);
une pluralité de zones de flasques saillantes (32, 34, 36) formées sur la surface interne (24) de l'élément cylindrique (12) et s'étendant de façon radiale dans le trou passant (14); caractérisée en ce que
une pluralité de rainures circonférentielles (26, 28, 30) sont espacées de façon longitudinale et en ce que chacune des zones de flasques saillantes (32, 34, 36) parmi la pluralité de flasques saillantes est située de façon à s'opposer de façon radiale à l'une des rainures respective (26, 28, 30).

2. La gaine pour la suture veineuse selon la Revendication 1, dans laquelle les zones de flasques saillantes (32, 34, 36) sont espacées angulairement autour de la surface interne (24) de l'élément cylindrique (12).

3. La gaine pour la suture veineuse selon la Revendication 1 ou la Revendication 2 ayant trois rainures (26, 28, 30) espacées de façon longitudinale.

4. La gaine pour la suture veineuse selon l'une des revendications précédentes, ayant trois zones de flasques saillantes (32, 34, 36) espacées le long de l'axe longitudinal (16) de l'élément cylindrique (12) et espacées forme d'angle autour de la surface interne (24) de l'élément cylindrique (12) à des intervalles d'environ 120 degrés.

5. La gaine pour la suture veineuse selon l'une des revendications précédentes, dans laquelle chacune desdites zones de flasques saillantes (32, 34, 36) a généralement une forme circulaire.

6. La gaine pour la suture veineuse selon l'une des revendications précédentes selon laquelle l'élément cylindrique (12) est formé d'un matériau élastique et en ce qu'elle comprend une fente (40) s'étendant de façon longitudinale de long de toute la longueur de l'élément cylindrique (12) et radialement au travers de l'épaisseur de l'élément cylindrique (12), l'élément cylindrique (12) pouvant s'étendre de façon élastique au niveau de la fente (40) pour entourer la veine (42) et le fil d'électrode inséré (44).

7. La gaine pour la suture veineuse selon l'une des revendications précédentes avec un diamètre interne plus grand que le diamètre externe de la veine (42) avec l'électrode insérée (44).

FIG. 1A
(PRIOR ART)

FIG. 1B
(PRIOR ART)

FIG. 2A
(PRIOR ART)

FIG. 2B
(PRIOR ART)

FIG. 3
(PRIOR ART)

EP 0 218 128 B1

**FIG. 4**

**FIG. 5**

α ≙ 120°

**FIG. 6**